(19) Europäisches Patentamt
European Patent Office
Office européen des brevets

(11) **EP 4 394 360 B1**

(12) **FASCICULE DE BREVET EUROPEEN**

(45) Date de publication et mention
de la délivrance du brevet:
**17.09.2025   Bulletin 2025/38**

(21) Numéro de dépôt: **23220209.3**

(22) Date de dépôt: **26.12.2023**

(51) Classification Internationale des Brevets (IPC):
**G01N 21/47** *(2006.01)*      **A61B 5/1455** *(2006.01)*
**G01N 21/49** *(2006.01)*

(52) Classification Coopérative des Brevets (CPC):
**G01N 21/474; A61B 5/14553; G01N 21/49;**
G01N 2021/4742

(54) **PROCÉDÉ D'ANALYSE D'UN OBJET COMPORTANT PLUSIEURS COUCHES SUPERPOSÈES, PAR MESURES DE RÉFLECTANCE OPTIQUE**

**VERFAHREN ZUR ANALYSE EINES OBJEKTS MIT MEHREREN SUPERPOSITIVEN SCHICHTEN DURCH OPTISCHE REFLEXIONSMESSUNGEN**

**METHOD FOR ANALYSING AN OBJECT HAVING SEVERAL SUPERPOSABLE LAYERS BY OPTICAL REFLECTANCE MEASUREMENTS**

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorité:   **27.12.2022   FR 2214565**

(43) Date de publication de la demande:
**03.07.2024   Bulletin 2024/27**

(73) Titulaire: **Commissariat à l'Energie Atomique et aux Energies Alternatives**
**75015 Paris (FR)**

(72) Inventeur: **PLANAT-CHRETIEN, Anne**
**38054 Grenoble cedex 09 (FR)**

(74) Mandataire: **INNOV-GROUP**
**209 Avenue Berthelot**
**69007 Lyon (FR)**

(56) Documents cités:
**US-A1- 2007 201 788      US-A1- 2015 018 642**
**US-A1- 2021 204 816      US-B2- 9 433 352**

**Description**

## DOMAINE TECHNIQUE

Le domaine technique de l'invention concerne l'analyse d'un objet comportant deux couches superposées, par mesures de réflectance optique.

## ART ANTERIEUR

La spectroscopie de réflectance diffuse, usuellement désignée par l'acronyme DRS (Diffuse Reflectance Spectroscopy), est une technique d'analyse non destructive permettant d'estimer des propriétés de propagation de la lumière dans un objet analysé. Cette technique est par exemple décrite dans EP2762064 ou EP3054282 ou EP3054281 ou EP3311138. Elle consiste à illuminer le milieu par un faisceau de lumière incident, et à détecter des photons rétrodiffusés par l'objet analysé, à distance du faisceau incident. Il est fréquent que la détection soit effectuée à différentes longueurs d'onde et ou à plusieurs distances du faisceau incident, de façon à obtenir des propriétés spectrales de propagation de la lumière dans l'objet analysé.

Le document US 9433352 B2 divulgue un dispositif de mesure optique qui mesure le degré d'absorbance de lumière d'un tissu de couche profonde tel que dans un corps humain ou un fruit.

Les propriétés de propagation de la lumière comprennent généralement des propriétés d'absorption et/ou des propriétés de diffusion de la lumière. Il s'agit notamment de coefficients d'absorption ou de diffusion, ces derniers représentant respectivement des probabilités d'absorption et de diffusion d'un photon par unité de longueur. L'estimation des propriétés de propagation de la lumière, à certaines longueurs d'onde, permet une estimation d'une concentration d'analytes dans l'objet analysé. Ainsi, la DRS peut être utilisée pour estimer les concentrations d'Oxyhémoglobine et déoxyhémoglobine par exemple - permettant le calcul du taux d'oxygénation du tissu et l'estimation de la quantité de hémoglobine totale (oxyhémogobline + désoxyhémoglobine).

Une difficulté peut survenir lorsque l'objet analysé n'est pas homogène, et comporte une couche superficielle sous laquelle s'étend une couche profonde. Afin de caractériser correctement la couche profonde, la contribution de la couche superficielle doit être prise en compte sous peine d'induire des erreurs d'estimation des concentrations d'analytes de la couche profonde. C'est d'autant plus vrai lorsque la couche superficielle évolue dans le temps : les changements de la couche superficielle peuvent être attribués à la couche profonde (ou réciproquement), ce qui peut induire de mauvais diagnostics ; lorsqu'il s'agit du compartiment extra-cérébral et cérébral par exemple, un faux négatif en cas d'hypoxie cérébrale peut avoir des conséquences cliniques et de prise en charge dramatiques.

Dans le cas de l'examen pratiqué sur la tête d'un individu, il est nécessaire de caractériser, de façon indépendante, une couche superficielle, correspondant à un compartiment extra-cérébral (peau, crane, dure-mère, liquide céphalorachidien), et d'une couche profonde, correspondant au cortex. Une telle caractérisation indépendante permet de distinguer la survenue d'une variation systémique de propriétés optiques, affectant simultanément les deux couches, de la survenue d'une variation cérébrale, affectant uniquement le cortex.

L'invention décrite ci-après répond à ce problème : il s'agit de séparer les contributions des couches superficielle et profonde de manière à estimer l'évolution des concentrations, en fonction du temps, d'un analyte dans les deux couches, l'analyte pouvant notamment être l'oxyhémoglobine ou la dé-oxyhémoglobine.

## EXPOSE DE L'INVENTION

Un premier objet de l'invention est un procédé de détermination d'une variation de propriétés d'absorption d'un objet, entre un premier instant et un deuxième instant, postérieur au premier instant, l'objet étant délimité par une surface, l'objet comportant une couche superficielle et une couche profonde, la couche superficielle s'étendant entre la surface et la couche profonde, le procédé comportant :

- a) illumination de l'objet par une source de lumière, la source de lumière émettant un faisceau d'illumination formant une zone d'illumination à la surface de l'objet ;
- b) détection de photons rétrodiffusés par l'objet, après s'être propagés dans l'objet, par un photodétecteur, les photons rétrodiffusés détectés émanant d'une zone de détection à la surface de l'objet, la zone de détection étant située à une distance de détection de la zone d'illumination, la distance de détection étant choisie parmi, dans un ordre croissant :

  • une première distance de détection, formant une première zone de détection ;
  • une deuxième distance de détection, formant une deuxième zone de détection ;
  • une troisième distance de détection, formant une troisième zone de détection ;

la détection des photons générant un signal de détection ;

le procédé étant caractérisé en ce qu'il comporte, chronologiquement, les étapes suivantes :

- (i) : au premier instant, mise en œuvre des étapes a) et b) en détectant, lors de l'étape b), les photons rétrodiffusés au niveau de la deuxième et de la troisième zones de détection ;
- (ii) : à partir des signaux de détection résultant de (i) :

  • prise en compte d'une propriété optique de diffusion dans l'objet ;
  • estimation d'un coefficient d'absorption de l'objet, la couche superficielle et la couche profonde étant considérées comme ayant le même coefficient d'absorption ;

- (iii) : à partir du coefficient d'absorption résultant de (ii), estimation d'une distance moyenne parcourue par les photons, dans la couche superficielle, entre la zone d'illumination et la première zone de détection;
- (iv) : au deuxième instant, mise en œuvre des étapes a) et b) en détectant, lors de l'étape b, les photons rétrodiffusés au niveau de la première zone de détection ;
- (v) : à partir du signal de détection résultant de (iv), et de la distance moyenne résultant de (iii), estimation d'une variation du coefficient d'absorption dans la couche superficielle entre le premier instant et le deuxième instant ;
- (vi) : au deuxième instant, mise en œuvre des étapes a) et b) en détectant, lors de l'étape b), les photons rétrodiffusés au niveau de la deuxième et de la troisième zones de détection ;
- (vii) : à partir des signaux de détection résultant de (vi), et de la variation du coefficient d'absorption dans la couche superficielle, résultant de (v), estimation du coefficient d'absorption dans la couche profonde au deuxième instant.

L'étape (i) peut comporter :

- à partir des signaux de détection mesurés au niveau de la deuxième zone de détection et de la troisième zone de détection, au premier instant, détermination d'une variation spatiale de l'absorbance de l'objet, au premier instant ;
- à partir de la variation spatiale de l'absorbance de l'objet, au premier instant, première estimation du coefficient d'absorption dans la couche superficielle et dans la couche profonde au premier instant ;
- application d'une première fonction de calibration d'absorption à la première estimation du coefficient d'absorption résultant de la sous-étape précédente, de façon à déterminer le coefficient d'absorption, dans la couche superficielle et dans la couche profonde, au premier instant.

L'étape (vii) peut comporter :

- à partir des signaux de détection mesurés au niveau de la deuxième zone de détection et de la troisième zone de détection, au deuxième instant, détermination d'une variation spatiale de l'absorbance de l'objet, au deuxième instant ;
- à partir de la variation spatiale de l'absorbance de l'objet, au deuxième instant, première estimation du coefficient d'absorption dans la couche profonde au deuxième instant ;
- application d'une deuxième fonction de calibration d'absorption à la première estimation du coefficient d'absorption résultant de la sous-étape précédente, de façon à déterminer le coefficient d'absorption, dans la couche profonde, au deuxième instant, la deuxième fonction de calibration d'absorption prenant en compte la variation du coefficient d'absorption dans la couche superficielle entre le premier instant et le deuxième instant.

L'étape (vii) peut comporter :

- à partir de la variation du coefficient d'absorption dans la couche superficielle résultant de (v), estimation d'une distance moyenne parcourue par les photons, dans la couche superficielle, entre la zone d'illumination et la première zone de détection, au deuxième instant ;
- calcul d'un ratio entre les distances moyennes parcourues par les photons résultant respectivement de la sous-étape précédente et de l'étape (ii) ;
- utilisation du ratio pour former la deuxième fonction de calibration d'absorption.

Selon une possibilité,

- le procédé comporte une estimation du coefficient d'absorption, dans la couche superficielle, au deuxième instant ;
- la deuxième fonction de calibration d'absorption est établie à l'aide de modélisations ou de mesures expérimentales effectuées sur des fantômes, chaque fantôme comportant :

- une couche superficielle, dont le coefficient d'absorption correspond au coefficient d'absorption estimé, dans la couche superficielle, au deuxième instant ;
- une couche profonde, dont le coefficient d'absorption est variable entre les différents fantômes.

Selon une possibilité

- la première distance de détection est inférieure à 2 cm ;
- les deuxième et troisième distances de détection sont supérieures à 2 cm.

Un deuxième objet de l'invention est un dispositif destiné à être appliqué face à une surface d'un objet entre au moins un premier instant et un deuxième instant, le dispositif comportant :

- une source de lumière configurée pour émettre un faisceau d'illumination, formant une zone d'illumination, à la surface de l'objet ;
- un photodétecteur, configuré pour former un signal de détection à partir d'une détection de photons rétrodiffusés par l'objet, au niveau ;

- d'une première zone de détection, s'étendant à une première distance de détection de la zone d'illumination ;
- d'une deuxième zone de détection, s'étendant à une deuxième distance de détection de la zone d'illumination, la deuxième distance de détection étant supérieure à la première distance de détection ;
- d'une troisième zone de détection , s'étendant à une troisième distance de détection de la zone d'illumination, la troisième distance de détection étant supérieure à la deuxième distance de détection ;

- une unité de traitement, programmée pour mettre en œuvre les étapes (ii), (iii), (v) et (vii) d'un procédé selon le premier objet de l'invention à partir de signaux de détection formés par le photodétecteur :

- lors de l'étape (ii), à partir de photons détectés au niveau des deuxième et troisième zone de détection ;
- lors l'étape (v), à partir de photons détectés au niveau de la première zone de détection ;
- lors de l'étape (vii), à partir de photons détectés au niveau des deuxième et troisième zones de détection.

La première distance de détection peut être inférieure à 2 cm. Les deuxième et troisième distances de détection peuvent être supérieures à 2 cm.

L'invention sera mieux comprise à la lecture de l'exposé des exemples de réalisation présentés, dans la suite de la description, en lien avec les figures listées ci-dessous.

## FIGURES

La figure 1A schématise un dispositif permettant une mise en œuvre de l'invention.
La figure 1B montre une zone d'illumination et une zone de détection formées à la surface d'un échantillon.
La figure 2 montre les principales étapes permettant une mise en œuvre de l'invention.
La figure 3 représente un exemple de fonction de calibration d'absorption.
La figure 4 montre un exemple de détermination d'une fonction de correction.
Les figures 5A à 5D montrent des résultats obtenus, par simulation, en mettant en œuvre l'invention selon une première configuration.
Les figures 6A à 6D montrent des résultats obtenus, par simulation, en mettant en œuvre l'invention selon une deuxième configuration.
Les figures 7A à 7D montrent des résultats obtenus, par simulation, en mettant en œuvre l'invention selon une troisième configuration.

## EXPOSE DE MODES DE REALISATION PARTICULIERS

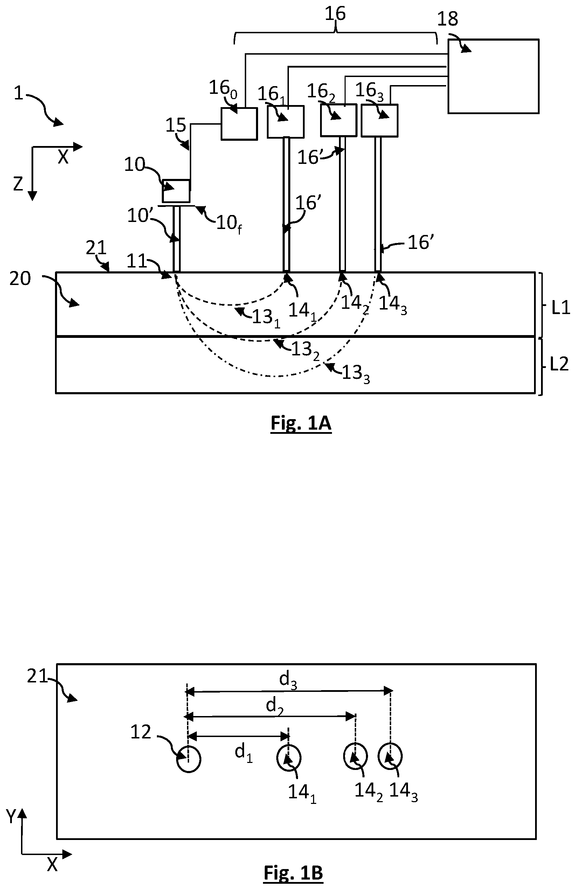

La figure 1A représente un dispositif 1 configuré pour estimer des propriétés optiques d'absorption dans un objet 20. Dans cet exemple, l'objet est un tissu biologique, par exemple une partie de la tête d'un individu, animal ou être humain.
Le dispositif comporte une source de lumière 10. La source de lumière est configurée pour émettre un faisceau lumineux 11, se propageant en direction de l'objet 20 à analyser. L'échantillon 20 est délimité par une surface 21. L'intersection du faisceau d'illumination 11 et de la surface 21 de l'échantillon forme une zone d'illumination 12, spatialement délimitée. La zone d'illumination est de préférence ponctuelle : elle est par exemple inscrite dans un cercle de diamètre inférieur à 1mm, voire inférieur à quelques centaines de $\mu$m, par exemple 100 $\mu$m ou 1 mm. La zone élémentaire d'illumination 12 est représentée sur la figure 1B.

La source de lumière peut être disposée au contact de l'objet 20 ou à distance de ce dernier. La source de lumière peut être un laser, une LED ou OLED (ou tout autre source de photons). Dans l'exemple représenté, la source de lumière est disposée à distance de l'échantillon. Le faisceau lumineux 11 est transporté jusqu'à la surface 21 de l'échantillon par une fibre optique d'illumination 10'. La source de lumière peut être filtrée en longueur d'onde par un filtre $10_f$.

Les photons formant le faisceau d'illumination 11 se propagent dans le tissu biologique à analyser. Le tissu biologique 20 est formé d'un milieu diffusant, susceptible d'absorber les photons, les propriétés de propagation des photons dans le milieu dépendant notamment de propriétés d'absorption ou de diffusion dans le milieu. De façon usuelle, les propriétés d'absorption peuvent être quantifiées par un coefficient d'absorption linéaire $\mu_a(\lambda)$. De façon connue, le coefficient d'absorption linéaire quantifie une probabilité d'absorption par le milieu par unité de longueur, à la longueur d'onde $\lambda$. Il est usuellement exprimé en cm$^{-1}$. Les propriétés de diffusion peuvent être quantifiées par un coefficient de diffusion $\mu_s(\lambda)$ ou un coefficient de diffusion réduit $\mu_s'(\lambda)$, qui quantifient une probabilité de diffusion par le milieu par unité de longueur, à la longueur d'onde $\lambda$. Il est usuellement exprimé en cm$^{-1}$.

Le dispositif comporte au moins trois photodétecteurs élémentaires $16_1$, $16_2$, $16_3$ formant un photodétecteur 16.Les photons détectés par le photodétecteur 16 émanent d'une zone élémentaire de détection 14 à la surface 21 de l'échantillon 20. La zone de détection 14 est de préférence ponctuelle, en étant, à l'instar de la zone d'illumination 12, inscrite dans un diamètre inférieur à 1 mm voire à 250 $\mu$m. La zone de détection 14 est séparée de la zone d'illumination 12. La distance entre la zone d'illumination 12 et la zone de détection 14 est une distance détection d. Elle peut être de quelques centimètres ou de l'ordre du centimètre, voire peut être inférieure à 1 cm.

Chaque photodétecteur peut être un ou plusieurs pixels d'un capteur d'image, un compteur de photons, un photodétecteur organique, une photodiode (ou tout autre composant permettant la détection de photons) Le dispositif est configuré pour former :

- une première zone de détection $14_1$, s'étendant à une première distance de détection d1 de la zone d'illumination ;
- une deuxième zone de détection $14_2$, s'étendant à une deuxième distance de détection d2 de la zone d'illumination, la deuxième distance de détection étant supérieure à la première distance de détection ;
- une troisième zone de détection $14_3$, s'étendant à une troisième distance de détection d3 de la zone d'illumination, la troisième distance de détection étant supérieure à la deuxième distance de détection.

Selon une possibilité, le nombre de zones de détection peut être supérieur à 3, en particulier lorsque le nombre de couches à caractériser est supérieur à 2.

Dans le cas d'une étude sur tête d'un fœtus ou d'un enfant, la première distance $d_1$ peut être inférieure à 1 cm. Les deuxième et troisième distances peuvent être supérieures à 1 cm, par exemple $d_2$ = 2.25 cm et $d_3$ = 2.5 cm.

Dans le cas d'une tête d'adulte, la première distance $d_1$ peut être de l'ordre de 1 cm. Les deuxième et troisième distances peuvent être supérieures ou égales à 3 cm, par exemple $d_2$ = 3 cm et $d_3$ = 3.5 cm.

Le choix des distances $d_1$, $d_2$ et $d_3$ dépend de l'épaisseur de la couche superficielle. La première distance $d_1$ est définir de façon que les photons rétrodiffusés dans la première zone de détection aient essentiellement traversé la couche superficielle. Les distances $d_2$ et $d_3$ sont choisies de façon à ce que la majorité des photons détectés aient traversé la couche profonde. On comprend que ces distances sont définies au cas par cas, selon la géométrie et les propriétés optiques de l'objet à caractériser. Ces distances dépendent donc des propriétés optiques et de l'épaisseur de la couche superficielle.

Sur la figure 1A, les trajets $13_1$, $13_2$ et $13_3$ correspondent à des trajets moyens parcourus par les photons respectivement détectés au niveau des zones de détection $14_1$, $14_2$, $14_3$.

Dans le mode de réalisation de l'invention, la source de lumière émet selon une longueur d'onde d'illumination pouvant correspondre à une longueur d'onde d'absorption de la désoxyhémoglobine ($\lambda$=750 nm) et/ou de l'oxyhémoglobine ($\lambda$=850 nm). Cela permet l'estimation de concentrations en désoxyhémoglobine et oxyhémoglobine à partir des coefficients d'absorption mesurés, selon des relations connues de l'homme du métier.

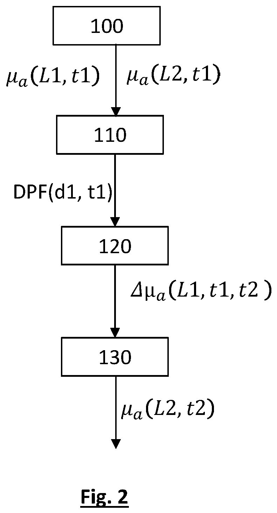

Le dispositif comporte une unité de traitement 18. L'unité de traitement comporte un microprocesseur programmé pour mettre en œuvre les étapes décrites par la suite, en lien avec la figure 2, à partir de signaux de détection détectés par le photodétecteur suite à une illumination de l'objet par la source de lumière.

Le dispositif est appliqué contre la surface 21 d'un objet 20, ce dernier comportant une couche superficielle L1 et une couche profonde L2. La couche superficielle est interposée entre la surface de l'objet et la couche profonde. Par couche, on entend une partie macroscopique de l'échantillon dans laquelle les propriétés optiques sont considérées comme homogènes.

L'objet 20 peut par exemple être un organe, par exemple une tête. Dans ce cas, la couche superficielle 21 correspond à une couche extra-cérébrale (peau+ graisse + crane+ dure-mère+ Liquide céphalo rachidien) et la couche profonde correspond à une couche cérébrale (cortex).

Il est connu qu'en utilisant des signaux de détection détectés au niveau de différentes zones de détection, on peut estimer les propriétés optiques, en particulier le coefficient d'absorption, d'un objet.

Par exemple, la technique de spectroscopie de réflectance diffuse (SRS - Spatially resolved spectroscopy), est une approche « multi distances », qui permet d'estimer un coefficient d'absorption, par exemple pour déterminer un niveau de saturation d'oxygène.

D'une façon générale, chaque mesure de réflectance consiste à mesurer le signal rétrodiffusé par le milieu, émanant d'une zone de détection située à une distance de détection de la zone d'illumination. On obtient ainsi un signal de détection $S(d, \lambda, t)$, dépendant du nombre de photons rétrodiffusés à la distance de détection. Le signal de détection est mesuré par le photodétecteur. Il s'agit cependant d'un signal de détection brut, qu'il est préférable de corriger.

Une première correction consiste à corriger l'offset (courant d'obscurité) de l'instrument, selon l'expression :

$$S_c(d, \lambda, t) = S(d, \lambda, t) - S_{offset}(\lambda) \ (1)$$

Où $S_{offset}(\lambda)$ est un signal d'obscurité, détecté alors que la source est éteinte. Cela correspond au bruit de détection associé à la chaine de mesure.

Une deuxième correction consiste à prendre en compte une dérive potentielle de la source de lumière 10, formant la zone d'illumination. Il s'agit de tenir compte d'une variation de la quantité de photons formant le faisceau d'illumination. Pour cela, une fibre de retour d'excitation 15 relie directement la source de lumière à un photodétecteur $16_0$. Le photodétecteur est ainsi configuré pour mesurer la quantité de lumière formant le faisceau d'illumination $S_{c,0}(\lambda, t)$ La réflectance correspond à un ratio entre $S_c(d, \lambda, t)$ et $S_{c,0}(\lambda, t)$ explicité selon l'expression :

$$R(d, \lambda, t) = \frac{S_c(d, \lambda, t)}{S_{c,0}(\lambda, t)} \ (2)$$

L'absorbance de l'objet, à la distance de détection d, est obtenue selon l'expression :

$$A(d, \lambda, t) = DO = -log_{10}\big(R(d, \lambda, t)\big) (3)$$

Selon l'approche « multi distances », on dispose de signaux de détection mesurés selon différentes distances de détection, par exemple d et d'. On obtient ainsi autant d'estimations de l'absorbance, à chaque distance de détection. Les coefficients $\mu_a$ et $\mu'_s$ sont reliés à l'absorbance par la relation :

$$\mu_a \mu'_s \approx \frac{1}{3}\Big(ln(10)\frac{\partial A}{\partial d} - \frac{2}{d}\Big)^2 (4)$$

avec

$$\frac{\partial A}{\partial d} \sim \frac{\Delta A}{\Delta d} = \frac{A(d, \lambda, t1) - A(d', \lambda, t1)}{d - d'} \ (5)$$

Si l'un des coefficients est connu, par exemple $\mu'_s$, on peut déduire $\mu_a$ de (4) et (5).

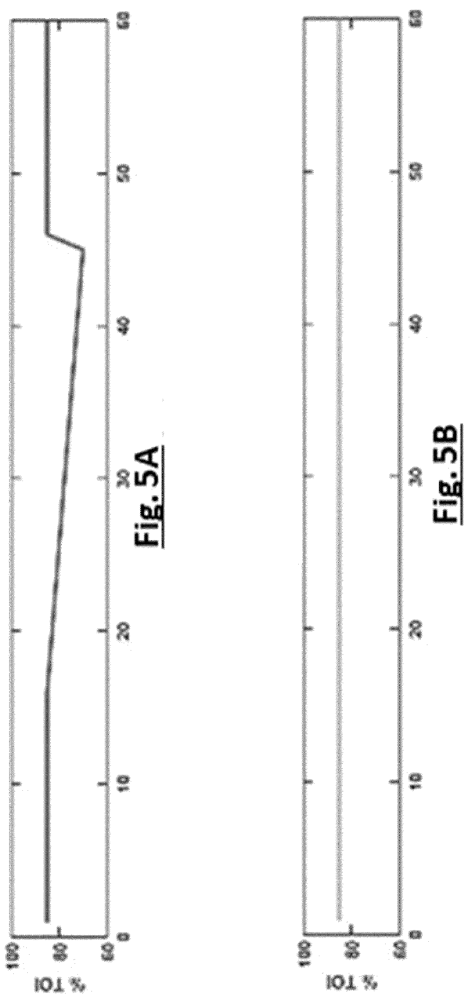

Une telle approche convient lorsque l'objet analysé est considéré comme homogène. Sur un objet non homogène, comportant une couche superficielle et une couche profonde, cette approche peut être appliquée pour déterminer le $\mu_a$ de la couche profonde, sous réserve que la couche superficielle soit suffisamment fine, typiquement inférieure à 0.5 cm ou 0.6 cm et les distances d2 et d3 suffisamment grandes Dans ce cas, la variation spatiale de l'absorbance $\Delta A$ permet de s'affranchir de la contribution de la couche superficielle , considérée comme identique en d2 et d3. En pratique (exemple de la tête d'enfant/adulte), cette correction ne permet pas de s'affranchir totalement de la contribution de la couche superficielle (voir exemple décrit en lien avec les figures 5A à 5D).

Selon une autre approche, dite MBL (Beer Lambert modifiée - Modified Beer Lambert), le coefficient d'absorption peut être estimé, à partir d'une absorbance $A(d)$ estimée selon une distance de détection d, par

$$A(d) = \frac{1}{ln(10)}(\mu_a d\, DPF + G) \quad (6),$$

où $DPF$ est un chemin moyen parcouru dans l'objet, par les photons formant le signal de détection

G est une constante dépendant de la diffusion du milieu et de la géométrie du dispositif. La constante G peut être éliminée en déterminant une variation temporelle de l'absorbance $\Delta A(t1, t2)$ entre deux instants t1 et t2.

$$\Delta A(t1, t2) = \frac{1}{ln(10)}\, d1\; DPF\; \Delta\mu_a(L1, t1, t2) \quad (7)$$

Où $\Delta\mu_a(L1, t1, t2)$ correspond à une variation de $\mu_a(L1)$ entre les instants t1 et t2 dans la couche superficielle L1.

La méthode MBL a déjà été mise en œuvre en combinant une courte distance de détection et une longue distance de détection, de façon à corriger la contribution de la couche superficielle dans l'estimation de l'absorption d'une couche profonde. Cependant, on a montré que cette méthode présente des limites, en particulier lorsque les variations temporelles du coefficient d'absorption sont de même type dans la couche profonde et dans la couche superficielle. Dans une telle situation, la couche profonde peut être « sur-corrigée » par la couche superficielle. De ce fait, une variation de l'absorption dans la couche profonde peut être masquée. Ainsi, en cas de survenue d'une hypoxie dans la couche superficielle et la couche profonde, l'hypoxie de la couche profonde peut être sous-estimée, voire non détectée.

L'approche MBL associée à la distance courte permet cependant de caractériser la couche superficielle de l'objet.

La figure 2 illustre les principales étapes de l'invention. Le dispositif utilisé permet de combiner les deux approches précédemment décrites (SRS et MBL), par le recours à une première distance de détection faible (i-e généralement < 1 cm) et deux distances de détection plus élevées (2 cm >d2>d3). Cela permet de combiner la méthode SRS en mettant en œuvre les signaux de détection détectés aux distances de détection $d_2$ et $d_3$, et la MBL, en mettant en œuvre un signal détecté à la distance de détection $d_1$.

Etape 100 : initialisation. Cette étape est effectuée à un premier instant t1
Au cours d'une étape 100, on prend en compte une hypothèse selon laquelle l'objet est homogène : la couche superficielle et la couche profonde sont considérées comme présentant les mêmes propriétés optiques de diffusion et d'absorption.

On prend en compte un coefficient de diffusion réduit $\mu s'$ déterminé a priori. Le document EP3054281 décrit une méthode permettant de déterminer le coefficient de diffusion réduit d'un milieu. Des modèles empiriques analytiques peuvent être utilisés.

Au cours de cette étape, on effectue une mesure de la réflectance selon deux distances de détection élevées, c'est-à-dire la deuxième distance d2 et la troisième distance d3.

A partir des signaux détectés $S(d2, \lambda, t1)$ et $S(d3, \lambda, t1)$, on met en œuvre les expressions (1) à (3) pour obtenir des absorbances $A(d2, \lambda, t1)$ et $A(d3, \lambda, t1)$.

$$\mu_a(\widetilde{L1}, t1)\mu'_s = \mu_a(\widetilde{L2}, t1)\mu'_s \approx \frac{1}{3}\left(ln(10)\frac{\partial A}{\partial d} - \frac{2}{d}\right)^2 (10),$$

dérivé de (4)
avec

$$\frac{\partial A}{\partial d} \sim \frac{\Delta A}{\Delta d} = \frac{A(d3, \lambda, t1) - A(d2, \lambda, t1)}{d3 - d2} \quad (11),$$

dérivé de (5)

$\mu_a(\widetilde{L1}, t1)$ est une estimation, au premier ordre, du coefficient d'absorption $\mu_a(L1, t1)$ de la couche superficielle L1, à l'instant t1 ;

$\mu_a(\widetilde{L2}, t1)$ est une estimation, au premier ordre, du le coefficient d'absorption $\mu_a(L2, t1)$ de la couche profonde L2, à l'instant t1 ;

Par hypothèse :

$$\mu_a(L1, t1) = \mu_a(L2, t1)$$

$\mu_a(\widetilde{L1, t1})$ et $\mu_a(\widetilde{L2, t1})$ sont déduits de l'expression (10) sachant que $\mu'_s$ est connu.

$\mu_a(L1, t1)$ et $\mu_a(L2, t1)$ et se déduisent de $\mu_a(\widetilde{L2, t1})$ et $\mu_a(\widetilde{L2, t1})$ en prenant en compte une première fonction de calibration d'absorption, établie sur la base de modélisations et/ou de fantômes de calibration en prenant en compte des coefficients $\mu_a$ et $\mu'_s$ connus. La fonction de calibration d'absorption permet de prendre en compte la réponse instrument, en particulier lorsque des fantômes connus sont utilisés, ou si la réponse instrument est prise en compte dans la modélisation.

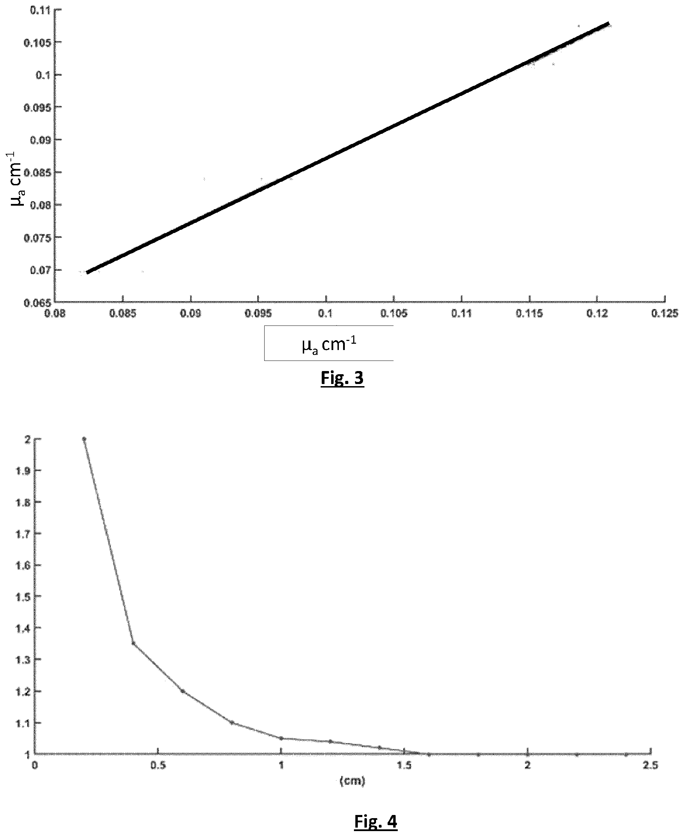

La figure 3 illustre un exemple de fonction de calibration d'absorption linéaire, de type :

$$\mu_a = a_0 \widetilde{\mu_a} + b_0 \quad (12).$$

En appliquant cette fonction à $\mu_a(\widetilde{L2, t1})$ (ou à $\mu_a(\widetilde{L2, t1})$), on obtient $\mu_a(L1, t1)$ et $\mu_a(L2, t1)$.

Sur la figure 3, l'axe des ordonnées correspond à la vraie valeur $\mu_a$ et l'axe des abscisses correspond à la valeur $\widetilde{\mu_a}$ résultant de la combinaison des expressions (10) et (11), à partir de signaux de détection mesurés sur fantômes homogènes ou modélisés. Par fantôme homogène, on entend un fantôme dont les propriétés optiques sont homogènes. Les scalaires $a_0$ et $b_0$ sont des paramètres de la fonction de calibration d'absorption.

Suite à l'étape 100, on dispose d'une estimation du coefficient $\mu_a$ de la couche superficielle L1 et de la couche profonde L2 à l'instant $t_1$.

Etape 110 : évaluation du chemin moyen des photons dans la couche superficielle, à l'instant t1.

Au cours de cette étape, on détermine le chemin moyen parcouru par les photons dans la couche superficielle, entre la zone d'illumination et la zone de détection correspondant à une distance de détection faible, c'est-à-dire la première distance de détection d1. La première distance de détection $d_1$ est choisie de façon que les photons détectés à cette distance soient essentiellement représentatifs de la couche superficielle. Sur la figure 1B, on a représenté le trajet moyen $13_1$ des photons détectés à la première distance de détection.

Le chemin moyen, noté DPF, acronyme de (Differential Pathlenght Factor), parcouru peut être estimé, en première approximation, selon l'expression :

$$\widehat{DPF}(d1, t1) = \frac{1}{2}\left(\frac{3\mu'_s}{\mu_a(L1,t1)}\right)^{1/2}\left(1 - \left(\frac{1}{1+d1(3\mu_a(L1,t1)\mu'_s)^{1/2}}\right)\right) \quad (13)$$

L'expression (13) a été décrite dans Scholkmann, F., & Wolf, M. General equation for the differential pathlength factor of the frontal human head depending on wavelength and age, 2013.

Cependant, l'expression analytique (13) est valable pour des grandes distances de détection, typiquement supérieures à 1 cm ou 2 cm. Or, la première distance de détection est généralement inférieure à 1 cm. Pour des distances de détection inférieures, il est possible d'appliquer un facteur de correction k, de telle sorte que

$$DPF(d1, t1) = k \times \widehat{DPF}(d1, t1) \quad (14)$$

La figure 4 montre l'évolution du facteur de recalage (axe des ordonnées) en fonction de la distance de détection (axe des abscisses - unité cm). La courbe de la figure 4 a été obtenue par simulations.

Etape 120 : détermination d'une variation du coefficient d'absorption de la couche superficielle à un instant t2

L'étape 120 est mise en œuvre à un deuxième instant t2, postérieur à l'instant t1. L'instant t2 peut être postérieur de quelques minutes ou quelques dizaines de minutes ou quelques heures à l'instant t1.

Au cours de cette étape, en utilisant le *DPF(d1, t1)* résultant de l'étape 110, on détermine le coefficient d'absorption de la première couche, à l'instant t2, selon l'expression :

$$A(d1, t2) = \frac{1}{ln(10)}(\mu_a(d1)d1 \, DPF(d1, t1) + G) \quad (15)$$

Comme précédemment décrit, en lien avec (6), la constante G peut être éliminée en déterminant une variation de l'absorbance $\Delta A(t1, t2)$ entre les instants t1 et t2.

$$\Delta A(t1, t2) = \frac{1}{ln(10)} \, d1 \; DPF \; \Delta\mu_a(L1, t1, t2) \quad (16)$$

Où $\Delta\mu_a(L1, t1, t2)$ correspond à une variation de $\mu_a(L1)$ entre les instants t1 et t2 dans la couche superficielle L1.

La mise en œuvre des expressions (15) et (16) suppose que la variation du *DPF* soit considérée comme négligeable entre les instants t1 et t2.

<u>Etape 130</u> mise à jour du coefficient d'absorption de la couche profonde à l'instant t2.

Cette étape vise à tenir compte de la variation du coefficient d'absorption, dans la couche superficielle, pour estimer le coefficient d'absorption dans la couche profonde à l'instant t2.

De même que dans l'étape 100, on effectue une mesure de la réflectance au niveau des distances de rétrodiffusion $d_2$ et $d_3$.

Ainsi,

$$\mu_a(\widetilde{L2}, t2)\mu'_s \approx \frac{1}{3}\left( ln(10)\frac{\partial A}{\partial d} - \frac{2}{d} \right)^2 (17)$$

avec

$$\frac{\partial A}{\partial d} \sim \frac{\Delta A}{\Delta d} = \frac{A(d3, \lambda, t2) - A(d2, \lambda, t2)}{d3 - d2} \quad (18)$$

L'expression (17) permet d'obtenir $\mu_a(\widetilde{L2}, t2)$, qui est une estimation au premier ordre du coefficient d'absorption $\mu_a(L2, t2)$ dans la couche profonde L2 à l'instant t2.

Selon la même approche que décrite dans l'étape 110, le recours à une fonction de calibration d'absorption, dite deuxième fonction de calibration d'absorption, prenant en compte la réponse instrument, est nécessaire de façon à déterminer $\mu_a(L2, t2)$. Un aspect important de l'invention est de prendre en compte que l'absorption dans la couche superficielle peut être différente de l'absorption dans la couche profonde (modèle bi-couche) pour établir la deuxième fonction de calibration d'absorption.

A l'instant t1, la fonction de calibration est linéaire, de type

$$\mu_a(L2, t1) = fa_0\mu_a(\widetilde{L2}, t1) + c_0 \quad (19)$$

où *f* et $c_0$ sont des scalaires.

Les paramètres $fa_0$ et $c_0$ sont déterminés sur la base de modélisations et/ou de mesures réalisées sur des fantômes bicouches, comportant une couche superficielle, dont le coefficient d'absorption est $\mu_a(L1, t1)$, et une couche profonde de la même épaisseur que l'objet analysé, et de préférence les mêmes propriétés de diffusion.

Or, d'après (12),

$$\mu_a(L2, t1) = a_0\mu_a(\widetilde{L2}, t1) + b_0 \quad (20)$$

En combinant (19) et (20), on obtient :

$$\mu_a(L2, t1) = fa_0\mu_a(\widetilde{L2}, t1) + (1-f)\mu_a(\widetilde{L2}, t1) + b_0 \quad (21)$$

L'expression (21) est une expression analytique, correspondant à une fonction de calibration d'absorption, à l'instant t1, permettant de déterminer $\mu_a(L2, t1)$ à partir de $\mu_a(\widetilde{L2}, t1)$, selon un modèle bicouche. Si elle est établie à partir d'essais expérimentaux, elle prend en compte la réponse instrument. Sinon, il est préférable d'introduire la réponse instrument dans la modélisation

A l'instant t2, l'expression (21) doit être corrigée, de façon à prendre en compte l'évolution de $\mu_a(L1)$ entre les instants t1 et t2.

A l'instant t2, la fonction de calibration d'absorption devient :

$$\mu_a(L2, t2) = gfa_0\mu_a(\widetilde{L2}, t2) + (1-f)\mu_a(\widetilde{L2}, t2) + b_0 \quad (21')$$

Avec

$$g = \frac{\widetilde{DPF}(d1,t2)}{\widetilde{DPF}(d1,t1)},$$

avec

$$\widetilde{DPF}(d1,t1) = \frac{1}{2}\left(\frac{3\mu_s'}{\mu_a(L1,t1)}\right)^{1/2}\left(1 - \left(\frac{1}{1+d1(3\mu_a(L1,t1)\mu_s')^{1/2}}\right)\right) (22)$$

$$\widetilde{DPF}(d1,t2) = \frac{1}{2}\left(\frac{3\mu_s'}{\mu_a(L1,t2)}\right)^{1/2}\left(1 - \left(\frac{1}{1+d1(3\mu_a(L1,t2)\mu_s')^{1/2}}\right)\right) (23)$$

Le terme $g$ traduit la prise en compte de l'évolution de l'absorption dans la couche superficielle entre les instants t1 et t2.

Ainsi, à partir de $\mu_a(\widetilde{L2,t2})$ résultant de (17), on applique (21), de façon à estimer $\mu_a(L2, t2)$, la variation de $\mu_a$ ($L1$), entre les instants t1 et t2, dans la couche superficielle L1 étant prise en compte dans le terme multiplicatif g.

Selon une autre possibilité, on détermine une fonction de correction, de type :

$$\mu_a(L2,t2) = a'_0 \mu_a(\widetilde{L2,t2}) + b'_0 \quad (24)$$

$a'_0$ et $b'_0$ étant déterminés à partir de modélisations et de mesures expérimentales sur des fantômes bicouches, le $\mu_a$ de la couche profonde étant variable, la couche superficielle ayant un coefficient d'absorption $\mu_a(L1, t2)$, tel que :

$$\mu_a(L1,t2) = \mu_a(L1,t1) + \Delta\mu_a(L1,t1,t2) \ (25)$$

Essais expérimentaux.

On a simulé une mise en œuvre de l'invention dans différentes configurations. Dans les configurations d'essai, on a pris en compte un tissu biologique, comportant une couche superficielle, d'épaisseur 0.5 mm, et une couche profonde et dont le coefficient de diffusion réduit était de 9.35cm$^{-1}$ à $\lambda$ = 750 nm et 7.64 cm-1 à $\lambda$ = 850 nm. On a simulé différents profils temporels du taux d'oxygénation (TOI) de la couche superficielle et de la couche profonde. TOI est un index d'oxygénation des tissus, tel que :

$$TOI = \frac{[HbO2]}{[HbO2]+[Hb]} \times 100 \quad (26)$$

[HbO2] correspond à la concentration d'oxyhémoglobine
[Hb] correspond à la concentration de déoxyhémoglobine.

L'invention a été mise en œuvre à un premier instant, qui correspond à t = 0, puis à différents instants successifs, jusqu'à t = 60. L'instant t1 correspond au premier instant. Les instants t2 correspondent à chaque instant de mesure jusqu'à t = 60. Les mesures simulées ont fait l'objet d'une addition d'un bruit : bruit photonique de type Poisson et bruit instrument de type gaussien.

L'invention a été mise en œuvre pour évaluer le coefficient d'absorption $\mu_a$, à $\lambda$=750 nm et à $\lambda$=850 nm, au cours du temps, dans la couche superficielle et dans la couche profonde. A partir de $\mu_a$, dans la couche superficielle et dans la couche profonde, on a estimé [HbO2] et [Hb] dans chacune de ces couches.

Selon une première configuration, cf. figures 5A à 5D, l'oxygénation de la couche superficielle varie (cf. figure 5A), tandis que l'oxygénation de la couche profonde est constante (cf. figure 5B). Sur les figures 5A et 5B, l'axe des abscisses correspond au temps (unité arbitraire) et l'axe des ordonnées correspond au taux d'oxygénation défini dans (26).

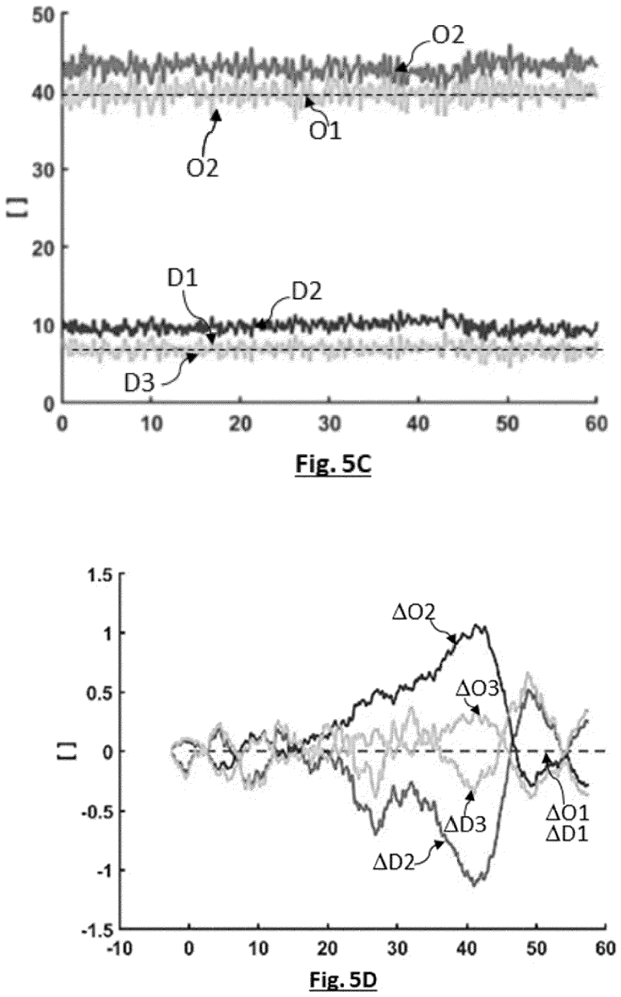

La figure 5C montre :

-  le profil réel de [HbO2] dans la couche profonde: cf. courbe O1

- les estimations de [HbO2] dans la couche profonde sans prise en compte de la variation de $\mu$a dans la couche superficielle par SRS: cf. courbe O2
- les estimations de [HbO2] dans la couche profonde en mettant en œuvre l'invention : cf. courbe 03
- le profil réel de [Hb] dans la couche profonde: cf. courbe D1
- les estimations de [Hb] dans la couche profonde sans prise en compte de la variation de $\mu$a dans la couche superficielle (méthode SRS dans cet exemple) : cf. courbe D2
- les estimations de [Hb] dans la couche profonde en mettant en œuvre l'invention : cf. courbe D3.

On observe que les courbes D3 et O3 sont plus proches des valeurs réelles D1 et O1.

La figure 5D montre :

- la variation réelle [HbO2] dans la couche profonde: cf. courbe $\Delta$O1
- les estimations de la variation de [HbO2] dans la couche profonde sans prise en compte de la variation de $\mu$a dans la couche superficielle : cf. courbe $\Delta$O2
- les estimations de la variation de [HbO2] dans la couche profonde en mettant en œuvre l'invention : cf. courbe $\Delta$O3
- la variation réelle de [Hb] dans la couche profonde: cf. courbe $\Delta$D1
- les estimations de la variation de [Hb] dans la couche profonde sans prise en compte de la variation de $\mu$a dans la couche superficielle : cf. courbe $\Delta$D2
- les estimations de la variation de [Hb] dans la couche profonde en mettant en œuvre l'invention : cf. courbe $\Delta$D3.

On observe que les courbes $\Delta$D3 et $\Delta$O3 sont plus proches des valeurs réelles $\Delta$D1 et $\Delta$O1.

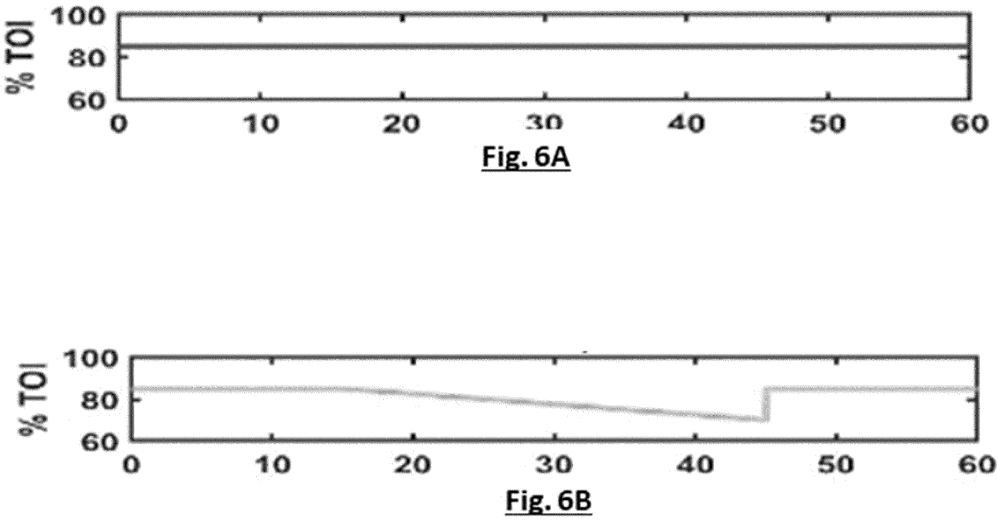

Selon une deuxième configuration, cf. figures 6A à 6D, l'oxygénation de la couche superficielle est stable (cf. figure 6A), tandis que l'oxygénation de la couche profonde varie (cf. figure 6B). Sur les figures 6A et 6B, l'axe des abscisses correspond au temps (unité arbitraire) et l'axe des ordonnées correspond au taux d'oxygénation défini dans (26).

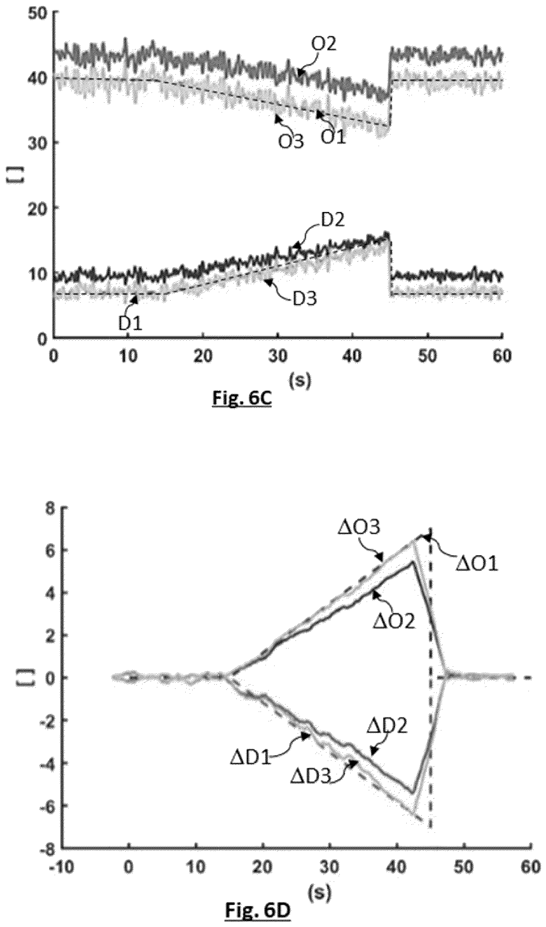

La figure 6C montre :

- le profil réel de [HbO2] dans la couche profonde: cf. courbe O1
- les estimations de [HbO2] dans la couche profonde sans prise en compte de la variation de $\mu$a dans la couche superficielle : cf. courbe O2
- les estimations de [HbO2] dans la couche profonde en mettant en œuvre l'invention : cf. courbe 03
- le profil réel de [Hb] dans la couche profonde: cf. courbe D1
- les estimations de [Hb] dans la couche profonde sans prise en compte de la variation de $\mu$a dans la couche superficielle : cf. courbe D2
- les estimations de [Hb] dans la couche profonde en mettant en œuvre l'invention : cf. courbe D3.

Sur la figure 6C, l'axe des abscisses correspond au temps (unité arbitraire) et l'axe des ordonnées correspond aux concentrations estimées.

On observe que les courbes D3 et O3 sont plus proches des valeurs réelles D1 et O1. Cela est dû au fait que l'invention permet une meilleure maîtrise de la concentration de Hb ou HbO2 dans la couche superficielle. Plus précisément, l'invention permet de prendre en compte la stabilité de l'absorption de la couche superficielle. Dans la méthode selon l'art antérieur, la variation de l'absorption dans la couche profonde est répartie à la fois dans la couche profonde et dans la couche superficielle.

La figure 6D montre :

- la variation réelle [HbO2] dans la couche profonde: cf. courbe $\Delta$O1
- les estimations de la variation de [HbO2] dans la couche profonde sans prise en compte de la variation de $\mu$a dans la couche superficielle : cf. courbe $\Delta$O2
- les estimations de la variation de [HbO2] dans la couche profonde en mettant en œuvre l'invention : cf. courbe $\Delta$O3
- la variation réelle de [Hb] dans la couche profonde: cf. courbe $\Delta$D1
- les estimations de la variation de [Hb] dans la couche profonde sans prise en compte de la variation de $\mu$a dans la couche superficielle : cf. courbe $\Delta$D2
- les estimations de la variation de [Hb] dans la couche profonde en mettant en œuvre l'invention : cf. courbe $\Delta$D3.

Sur la figure 6D, l'axe des abscisses correspond au temps (unité arbitraire) et l'axe des ordonnées correspond aux variations de concentrations estimées.

On observe que les courbes $\Delta$D3 et $\Delta$O3 sont plus proches des valeurs réelles $\Delta$D1 et $\Delta$O1.

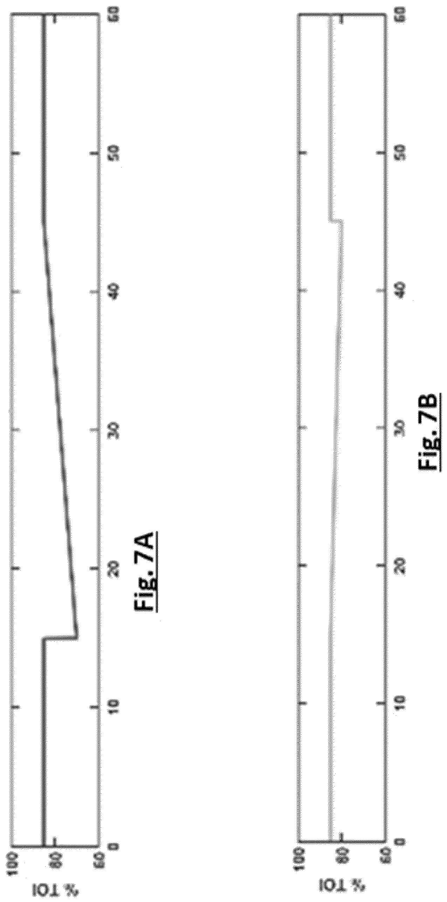

Selon une troisième configuration, cf. figures 7A à 7D, l'oxygénation de la couche superficielle et l'oxygénation de la couche profonde varient (cf. figures 7A et 7B). Sur les figures 7A et 7B, l'axe des abscisses correspond au temps (unité arbitraire) et l'axe des ordonnées correspond au taux d'oxygénation défini dans (26).

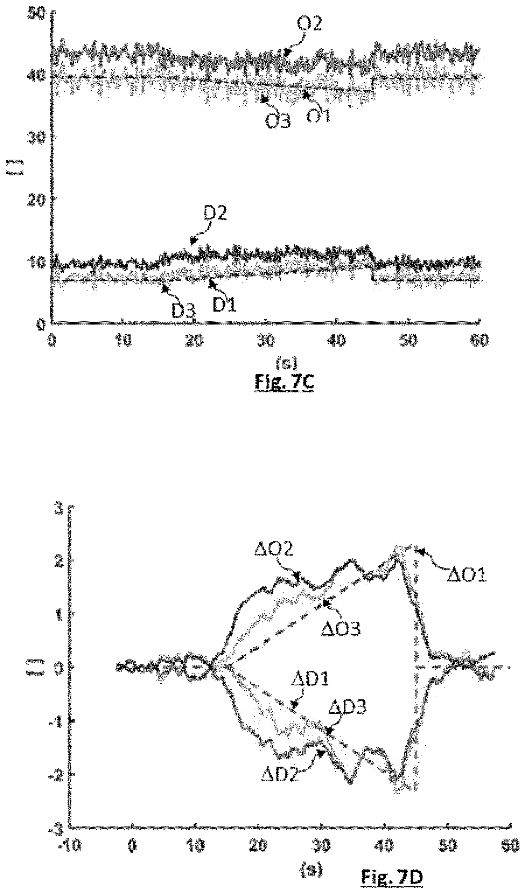

La figure 7C montre :

- le profil réel de [HbO2] dans la couche profonde: cf. courbe O1
- les estimations de [HbO2] dans la couche profonde sans prise en compte de la variation de μa dans la couche superficielle : cf. courbe O2
- les estimations de [HbO2] dans la couche profonde en mettant en œuvre l'invention : cf. courbe 03
- le profil réel de *[Hb]* dans la couche profonde: cf. courbe D1
- les estimations de *[Hb]* dans la couche profonde sans prise en compte de la variation de μa dans la couche superficielle : cf. courbe D2
- les estimations de *[Hb]* dans la couche profonde en mettant en œuvre l'invention : cf. courbe D3.

Sur la figure 7C, l'axe des abscisses correspond au temps (unité arbitraire) et l'axe des ordonnées correspond aux concentrations estimées.

On observe que les courbes D3 et O3 sont plus proches des valeurs réelles D1 et O1.

La figure 7D montre :

- la variation réelle [HbO2] dans la couche profonde: cf. courbe $\Delta$O1
- les estimations de la variation de [HbO2] dans la couche profonde sans prise en compte de la variation de μa dans la couche superficielle : cf. courbe $\Delta$O2
- les estimations de la variation de [HbO2] dans la couche profonde en mettant en œuvre l'invention : cf. courbe $\Delta$O3
- la variation réelle de *[Hb]* dans la couche profonde: cf. courbe $\Delta$D1
- les estimations de la variation de *[Hb]* dans la couche profonde sans prise en compte de la variation de μa dans la couche superficielle : cf. courbe $\Delta$D2
- les estimations de la variation de *[Hb]* dans la couche profonde en mettant en œuvre l'invention : cf. courbe $\Delta$D3.

Sur la figure 7D, l'axe des abscisses correspond au temps (unité arbitraire) et l'axe des ordonnées correspond aux variations de concentrations estimées.

On observe que les courbes $\Delta$D3 et $\Delta$O3 sont plus proches des valeurs réelles $\Delta$D1 et $\Delta$O1.


**Revendications**

1. Procédé de détermination d'une variation de propriétés d'absorption d'un objet (20), entre un premier instant (t1) et un deuxième instant (t2), postérieur au premier instant, l'objet étant délimité par une surface (21), l'objet comportant une couche superficielle (L1) et une couche profonde (L2), la couche superficielle s'étendant entre la surface et la couche profonde, le procédé comportant :

   - a) illumination de l'objet par une source de lumière (10), la source de lumière émettant un faisceau d'illumination (11) formant une zone (12) à la surface de l'objet ;
   - b) détection de photons rétrodiffusés par l'objet, après s'être propagés dans l'objet, par un photodétecteur (16), les photons rétrodiffusés détectés émanant d'une zone de détection ($14_1$, $14_2$, $14_3$) à la surface de l'objet, la zone de détection étant située à une distance de détection de la zone d'illumination, la distance de détection étant choisie parmi, dans un ordre croissant :

      • une première distance de détection (d1), formant une première zone de détection ;
      • une deuxième distance de détection (d2) formant une deuxième zone de détection ;
      • une troisième distance de détection (d3), formant une troisième zone de détection ;

   la détection des photons générant un signal de détection ;
   le procédé étant **caractérisé en ce qu'**il comporte, chronologiquement, les étapes suivantes :

      - (i) : au premier instant, mise en œuvre des étapes a) et b) en détectant les photons rétrodiffusés au niveau de la deuxième et de la troisième zones de détection ;
      - (ii) : à partir des signaux de détection résultant de (i) :

         • prise en compte d'une propriété optique de diffusion dans l'objet ;
         • estimation d'un coefficient d'absorption de l'objet, la couche superficielle et la couche profonde étant considérées comme ayant le même coefficient d'absorption ($\mu_a$(L1, t1),$\mu_a$(L2, t1));

- (iii) : à partir du coefficient d'absorption résultant de (ii), estimation d'une distance moyenne parcourue par les photons (DPF(d1, t1)), dans la couche superficielle, entre la zone d'illumination et la première zone de détection;

- (iv) : au deuxième instant, mise en œuvre des étapes a) et b) en détectant les photons rétrodiffusés au niveau de la première zone de détection ;

- (v) : à partir du signal de détection résultant de (iv), et de la distance moyenne résultant de (iii), estimation d'une variation du coefficient d'absorption ($\Delta\mu_a$(L1, t1, t2 )) dans la couche superficielle entre le premier instant et le deuxième instant ;

- (vi) : au deuxième instant, mise en œuvre des étapes a) et b) en détectant, lors de l'étape b), les photons rétrodiffusés au niveau de la deuxième et de la troisième zones de détection ;

- (vii) : à partir des signaux de détection résultant de (vi), et de la variation du coefficient d'absorption dans la couche superficielle, résultant de (v), estimation du coefficient d'absorption dans la couche profonde au deuxième instant ($\mu_a$(L2, t2)).

2. Procédé selon la revendication 1, dans lequel l'étape (i) comporte :

• à partir des signaux de détection mesurés au niveau de la deuxième zone de détection et de la troisième zone de détection, au premier instant, détermination d'une variation spatiale de l'absorbance de l'objet ($\Delta A$) au premier instant (t1);

• à partir de la variation spatiale de l'absorbance de l'objet, au premier instant, première estimation du coefficient d'absorption dans la couche superficielle ( $\mu_a(\widetilde{L1, t1})$ )et dans la couche profonde $\mu_a(\widetilde{L2, t1})$ au premier instant ;

• application d'une première fonction de calibration d'absorption à la première estimation du coefficient d'absorption résultant de la sous-étape précédente, de façon à déterminer le coefficient d'absorption ($\mu_a$(L1, t1),$\mu_a$(L2, t1)), dans la couche superficielle et dans la couche profonde, au premier instant.

3. Procédé selon la revendication 2, dans l'étape (vii) comporte :

• à partir des signaux de détection mesurés au niveau de la deuxième zone de détection et de la troisième zone de détection, au deuxième instant, détermination d'une variation spatiale de l'absorbance de l'objet ($\Delta A$), au deuxième instant (t2);

• à partir de la variation spatiale de l'absorbance de l'objet, au deuxième instant, première estimation du coefficient d'absorption dans la couche profonde au deuxième instant ( $\mu_a(\widetilde{L2, t2})$ );

• application d'une deuxième fonction de calibration d'absorption à la première estimation du coefficient d'absorption résultant de la sous-étape précédente, de façon à déterminer le coefficient d'absorption, dans la couche profonde, au deuxième instant ($\mu_a$(L2, t2)), la deuxième fonction de calibration d'absorption prenant en compte la variation du coefficient d'absorption dans la couche superficielle entre le premier instant et le deuxième instant.

4. Procédé selon la revendication 3, dans lequel l'étape (vii) comporte :

- à partir de la variation du coefficient d'absorption dans la couche superficielle résultant de (v), estimation d'une distance moyenne parcourue par les photons, dans la couche superficielle, entre la zone d'illumination et la première zone de détection, au deuxième instant ;

- calcul d'un ratio (g) entre les distances moyennes parcourues par les photons résultant respectivement de la sous-étape précédente et de l'étape (ii) ;

- utilisation du ratio pour former la deuxième fonction de calibration d'absorption.

5. Procédé selon la revendication 3 dans lequel

- le procédé comporte une estimation du coefficient d'absorption, dans la couche superficielle, au deuxième instant ;

- la deuxième fonction de calibration d'absorption est établie à l'aide de modélisations ou de mesures expérimentales effectuées sur des fantômes, chaque fantôme comportant :

• une couche superficielle, dont le coefficient d'absorption correspond au coefficient d'absorption estimé, dans la couche superficielle, au deuxième instant ;
• une couche profonde, dont le coefficient d'absorption est variable entre les différents fantômes.

6. Procédé selon l'une quelconque des revendications précédentes, dans lequel

- la première distance de détection est inférieure à 2 cm ;
- les deuxième et troisième distances de détection sont supérieures à 2 cm.

7. Dispositif (1) destiné à être appliqué face à une surface d'un objet entre au moins un premier instant et un deuxième instant, le dispositif comportant :

- une source de lumière (10) configurée pour émettre un faisceau d'illumination (11), formant une zone d'illumination (12), à la surface de l'objet ;
- un photodétecteur, configuré pour former un signal de détection à partir d'une détection de photons rétrodiffusés par l'objet, au niveau ;

• d'une première zone de détection ($14_1$), s'étendant à une première distance de détection (d1) de la zone d'illumination ;
• d'une deuxième zone de détection ($14_2$), s'étendant à une deuxième distance de détection (d2) de la zone d'illumination, la deuxième distance de détection étant supérieure à la première distance de détection ;
• d'une troisième zone de détection ($14_3$), s'étendant à une troisième distance (d3) de détection de la zone d'illumination, la troisième distance de détection étant supérieure à la deuxième distance de détection ;

- une unité de traitement (18), programmée pour mettre en œuvre les étapes (ii), (iii), (v) et (vii) d'un procédé selon l'une quelconque des revendications précédentes à partir de signaux de détection formés par le photodétecteur :

• lors de l'étape (ii), à partir de photons détectés au niveau des deuxième et troisième zone de détection ;
• lors l'étape (v), à partir de photons détectés au niveau de la première zone de détection ;
• lors de l'étape (vii), à partir de photons détectés au niveau des deuxième et troisième zones de détection.

8. Dispositif selon la revendication 7, dans lequel

- la première distance de détection est inférieure à 2 cm ;
- les deuxième et troisième distances de détection sont supérieures à 2 cm.

**Patentansprüche**

1. Verfahren zur Bestimmung einer Änderung von Absorptionseigenschaften eines Objekts (20) zwischen einem ersten Zeitpunkt (t1) und einem zweiten Zeitpunkt (t2) nach dem ersten Zeitpunkt, wobei das Objekt durch eine Oberfläche (21) begrenzt wird, wobei das Objekt eine oberflächliche Schicht (L1) und eine tiefe Schicht (L2) umfasst, wobei sich die oberflächliche Schicht zwischen der Oberfläche und der tiefen Schicht erstreckt, wobei das Verfahren umfasst:

- a) Beleuchten des Objekts durch eine Lichtquelle (10), wobei die Lichtquelle einen Beleuchtungsstrahl (11) emittiert, der einen Bereich (12) an der Oberfläche des Objekts bildet;
- b) Detektion von Photonen, die von dem Objekt rückgestreut werden, nachdem sie sich in dem Objekt ausgebreitet haben, durch einen Fotodetektor (16), wobei die detektierten rückgestreuten Photonen von einem Detektionsbereich ($14_1$, $14_2$, $14_3$) an der Oberfläche des Objekts ausgehen, wobei der Detektionsbereich in einer Detektionsdistanz von dem Beleuchtungsbereich gelegen ist, wobei die Detektionsdistanz, in einer aufsteigenden Reihenfolge, ausgewählt ist aus:

• einer ersten Detektionsdistanz (d1), die einen ersten Detektionsbereich bildet;
• einer zweiten Detektionsdistanz (d2), die einen zweiten Detektionsbereich bildet;
• einer dritten Detektionsdistanz (d3), die einen dritten Detektionsbereich bildet;

wobei die Detektion der Photonen ein Detektionssignal erzeugt;
wobei das Verfahren **dadurch gekennzeichnet ist, dass** es, chronologisch, die folgenden Schritte umfasst:

- (i): zu dem ersten Zeitpunkt, Durchführen der Schritte a) und b), indem die rückgestreuten Photonen an dem zweiten und dem dritten Detektionsbereich detektiert werden;
- (ii): ausgehend von den aus (i) resultierenden Detektionssignalen:

  • Berücksichtigen einer optischen Streueigenschaft in dem Objekt;
  • Schätzen eines Absorptionskoeffizienten des Objekts, wobei die oberflächliche Schicht und die tiefe Schicht als denselben Absorptionskoeffizienten ($\mu_a$ (L1,t1), $\mu_a$ (L2,t1)) aufweisend betrachtet werden;

- (iii): ausgehend von dem aus (ii) resultierenden Absorptionskoeffizienten, Schätzen einer von den Photonen zurückgelegten mittleren Distanz (DPF (d1,t1)) in der oberflächlichen Schicht zwischen dem Beleuchtungsbereich und dem ersten Detektionsbereich;
- (iv): zu dem zweiten Zeitpunkt, Durchführen der Schritte a) und b), indem die rückgestreuten Photonen an dem ersten Detektionsbereich detektiert werden;
- (v): ausgehend von dem aus (iv) resultierenden Detektionssignal und von der aus (iii) resultierenden mittleren Distanz, Schätzen einer Änderung des Absorptionskoeffizienten ($\Delta\mu_a$(L1, t1, t2 )) in der oberflächlichen Schicht zwischen dem ersten Zeitpunkt und dem zweiten Zeitpunkt;
- (vi): zu dem zweiten Zeitpunkt, Durchführen der Schritte a) und b), indem, bei dem Schritt b), die rückgestreuten Photonen an dem zweiten und dem dritten Detektionsbereich detektiert werden;
- (vii): ausgehend von den aus (vi) resultierenden Detektionssignalen und der aus (v) resultierenden Änderung des Absorptionskoeffizienten in der oberflächlichen Schicht, Schätzen des Absorptionskoeffizienten in der tiefen Schicht zu dem zweiten Zeitpunkt ($\mu_a$(L2, t2)).

2. Verfahren nach Anspruch 1, wobei der Schritt (i) umfasst:

  • ausgehend von den Detektionssignalen, die an dem zweiten Detektionsbereich und dem dritten Detektionsbereich zu dem ersten Zeitpunkt gemessen werden, Bestimmen einer räumlichen Änderung des Absorptionsgrades des Objekts ($\Delta A$) zu dem ersten Zeitpunkt (t1);
  • ausgehend von der räumlichen Änderung des Absorptionsgrades des Objekts zu dem ersten Zeitpunkt, erstes Schätzen des Absorptionskoeffizienten in der oberflächlichen Schicht ($\mu_a\left(\widetilde{L1}, t1\right)$) und in der tiefen Schicht $\mu_a\left(\widetilde{L2}, t1\right)$ zu dem ersten Zeitpunkt;
  • Anwenden einer ersten Absorptionskalibrierungsfunktion auf die aus dem vorhergehenden Teilschritt resultierende erste Schätzung des Absorptionskoeffizienten, so dass der Absorptionskoeffizient ($\mu_a$ (L1, t1), $\mu_a$ (L2,t1)) in der oberflächlichen Schicht und in der tiefen Schicht zu dem ersten Zeitpunkt bestimmt wird.

3. Verfahren nach Anspruch 2, wobei der Schritt (vii) umfasst:

  • ausgehend von den Detektionssignalen, die an dem zweiten Detektionsbereich und dem dritten Detektionsbereich zu dem zweiten Zeitpunkt gemessen werden, Bestimmen einer räumlichen Änderung des Absorptionsgrades des Objekts ($\Delta A$) zu dem zweiten Zeitpunkt (t2);
  • ausgehend von der räumlichen Änderung des Absorptionsgrades des Objekts zu dem zweiten Zeitpunkt, erstes Schätzen des Absorptionskoeffizienten in der tiefen Schicht zu dem zweiten Zeitpunkt ($\mu_a\left(\widetilde{L2}, t2\right)$);
  • Anwenden einer zweiten Absorptionskalibrierungsfunktion auf die aus dem vorhergehenden Teilschritt resultierende erste Schätzung des Absorptionskoeffizienten, so dass der Absorptionskoeffizient in der tiefen Schicht zu dem zweiten Zeitpunkt ($\mu_a$ (L2, t2)) bestimmt wird, wobei die zweite Absorptionskalibrierungsfunktion die Änderung des Absorptionskoeffizienten in der oberflächlichen Schicht zwischen dem ersten Zeitpunkt und dem zweiten Zeitpunkt berücksichtigt.

4. Verfahren nach Anspruch 3, wobei der Schritt (vii) umfasst:

  - ausgehend von der aus (v) resultierenden Änderung des Absorptionskoeffizienten in der oberflächlichen Schicht, Schätzen einer von den Photonen zurückgelegten mittleren Distanz in der oberflächlichen Schicht zwischen dem Beleuchtungsbereich und dem ersten Detektionsbereich zu dem zweiten Zeitpunkt;
  - Berechnen eines Verhältnisses (g) zwischen den aus dem vorhergehenden Teilschritt und dem Schritt (ii) resultierenden von den Photonen zurückgelegten mittleren Distanzen;
  - Verwenden des Verhältnisses zum Bilden der zweiten Absorptionskalibrierungsfunktion.

**5.** Verfahren nach Anspruch 3, wobei

- das Verfahren ein Schätzen des Absorptionskoeffizienten in der oberflächlichen Schicht zu dem zweiten Zeitpunkt umfasst;
- die zweite Absorptionskalibrierungsfunktion mithilfe von Modellierungen oder experimentellen Messungen, die an Phantomen durchgeführt werden, erstellt wird, wobei jedes Phantom umfasst:

  • eine oberflächliche Schicht, deren Absorptionskoeffizient dem in der oberflächlichen Schicht zu dem zweiten Zeitpunkt geschätzten Absorptionskoeffizienten entspricht;
  • eine tiefe Schicht, deren Absorptionskoeffizient zwischen den verschiedenen Phantomen variabel ist.

**6.** Verfahren nach einem der vorhergehenden Ansprüche, wobei:

- die erste Detektionsdistanz weniger als 2 cm beträgt;
- die zweite und die dritte Detektionsdistanz mehr als 2 mm betragen.

**7.** Vorrichtung (1), die dazu bestimmt ist, gegenüber einer Oberfläche eines Objekts zwischen mindestens einem ersten Zeitpunkt und einem zweiten Zeitpunkt angebracht zu werden, wobei die Vorrichtung umfasst:

- eine Lichtquelle (10), die dazu ausgestaltet ist, einen Beleuchtungsstrahl (11) zu emittieren, der einen Beleuchtungsbereich (12) an der Oberfläche des Objekts bildet;
- einen Fotodetektor, der dazu ausgestaltet ist, ein Detektionssignal ausgehend von einer Detektion von Photonen zu bilden, die von dem Objekt rückgestreut werden, an:

  • einem ersten Detektionsbereich ($14_1$), der sich in einer ersten Detektionsdistanz (d1) von dem Beleuchtungsbereich erstreckt;
  • einem zweiten Detektionsbereich ($14_2$), der sich in einer zweiten Detektionsdistanz (d2) von dem Beleuchtungsbereich erstreckt, wobei die zweite Detektionsdistanz größer als die erste Detektionsdistanz ist;
  • einem dritten Detektionsbereich ($14_3$), der sich in einer dritten Detektionsdistanz (d3) von dem Beleuchtungsbereich erstreckt, wobei die dritte Detektionsdistanz größer als die zweite Detektionsdistanz ist;

- eine Verarbeitungseinheit (18), die dazu programmiert ist, die Schritte (ii), (iii), (v) und (vii) eines Verfahrens nach einem der vorhergehenden Ansprüche ausgehend von Detektionssignalen durchzuführen, die von dem Fotodetektor gebildet werden:

  • bei dem Schritt (ii) ausgehend von Photonen, die an dem zweiten und dem dritten Detektionsbereich detektiert werden;
  • bei dem Schritt (v) ausgehend von Photonen, die an dem ersten Detektionsbereich detektiert werden;
  • bei dem Schritt (vii) ausgehend von Photonen, die an dem zweiten und dem dritten Detektionsbereich detektiert werden.

**8.** Vorrichtung nach Anspruch 7, wobei

- die erste Detektionsdistanz weniger als 2 cm beträgt;
- die zweite und die dritte Detektionsdistanz mehr als 2 mm betragen.

## Claims

**1.** Method for determining a variation of absorption properties of an object (20), between a first instant (t1) and a second instant (t2) later than the first instant, the object being delimited by a surface (21), the object comprising a surface layer (L1) and a deep layer (L2), the surface layer extending between the surface and the deep layer, the method comprising:

- a) illumination of the object by a light source (10), the light source emitting an illumination beam (11) forming a zone (12) on the surface of the object;
- b) detection of photons backscattered by the object, after being propagated in the object, by a photodetector (16), the detected backscattered photons emanating from a detection zone ($14_1$, $14_2$, $14_3$) on the surface of the object, the detection zone being situated at a detection distance from the illumination zone, the detection distance being chosen from among, in ascending order:

- a first detection distance (d1), forming a first detection zone;
- a second detection distance (d2), forming a second detection zone;
- a third detection distance (d3), forming a third detection zone;
the detection of the photons generating a detection signal;
the method being **characterized in that** it comprises, chronologically, the following steps:

- (i): at the first instant, implementation of the steps a) and b) by detecting the photons backscattered in the second and third detection zones;
- (ii): from the detection signals resulting from (i):

- recognition of an optical property of diffusion in the object;
- estimation of an absorption coefficient of the object, the surface layer and the deep layer being considered as having the same absorption coefficient ($\mu_a$ (L1,t1), $\mu_a$ (L2,t1));

- (iii): from the absorption coefficient resulting from (ii), estimation of an average distance travelled by the photons *(DPF (d1,t1))*, in the surface layer, between the illumination zone and the first detection zone;
- (iv): at the second instant, implementation of the steps a) and b) by detecting the photons backscattered in the first detection zone;
- (v): from the detection signal resulting from (iv), and the average distance resulting from (iii), estimation of a variation of the absorption coefficient ($\Delta\mu_a$(L1, t1, t2 )) in the surface layer between the first instant and the second instant;
- (vi): at the second instant, implementation of the steps a) and b) by detecting, in the step b), the photons backscattered in the second and third detection zones;
- (vii): from the detection signals resulting from (vi), and the variation of the absorption coefficient in the surface layer, resulting from (v), estimation of the absorption coefficient in the deep layer at the second instant ($\mu_a$(L2, t2)).

2. Method according to Claim 1, wherein the step (i) comprises:

- from the detection signals measured in the second detection zone and the third detection zone, at the first instant, determination of a spatial variation of the absorbance of the object ($\Delta A$) at the first instant *(t1);*
- from the spatial variation of the absorbance of the object, at the first instant, first estimation of the absorption coefficient in the surface layer ( $\mu_a\left(\widetilde{L1}, t1\right)$ ) and in the deep layer $\mu_a\left(\widetilde{L2}, t1\right)$ at the first instant;
- application of a first absorption calibration function to the first estimation of the absorption coefficient resulting from the preceding substep, so as to determine the absorption coefficient ($\mu_a$ (L1, t1), $\mu_a$ (L2,t1)), in the surface layer and in the deep layer, at the first instant.

3. Method according to Claim 2, wherein the step (vii) comprises:

- from the detection signals measured in the second detection zone and the third detection zone, at the second instant, determination of a spatial variation of the absorbance of the object ($\Delta A$), at the second instant *(t2);*
- from the spatial variation of the absorbance of the object, at the second instant, first estimation of the absorption coefficient in the deep layer at the second instant ( $\mu_a\left(\widetilde{L2}, t2\right)$ );
- application of a second absorption calibration function to the first estimation of the absorption coefficient resulting from the preceding substep, so as to determine the absorption coefficient, in the deep layer, at the second instant ($\mu_a$ (L2, t2)), the second absorption calibration function taking into account the variation of the absorption coefficient in the surface layer between the first instant and the second instant.

4. Method according to Claim 3, wherein the step (vii) comprises:

- from the variation of the absorption coefficient in the surface layer resulting from (v), estimation of an average distance travelled by the photons, in the surface layer, between the illumination zone and the first detection zone, at the second instant;

- calculation of a ratio (g) between the average distances travelled by the photons resulting respectively from the preceding substep and the step (ii);
- use of the ratio to form the second absorption calibration function.

5. Method according to Claim 3, wherein

- the method comprises an estimation of the absorption coefficient, in the surface layer, at the second instant;
- the second absorption calibration function is established using modellings or experimental measurements performed on phantoms, each phantom comprising:

   • a surface layer, the absorption coefficient of which corresponds to the absorption coefficient estimated, in the surface layer, at the second instant;
   • a deep layer, the absorption coefficient of which is variable between the different phantoms.

6. Method according to any one of the preceding claims, wherein

- the first detection distance is less than 2 cm;
- the second and third detection distances are greater than 2 cm.

7. Device (1) intended to be applied facing a surface of an object between at least one first instant and a second instant, the device comprising:

- a light source (10) configured to emit an illumination beam (11), forming an illumination zone (12), on the surface of the object;
- a photodetector, configured to form a detection signal from a detection of photons backscattered by the object, in:

   • a first detection zone ($14_1$), extending to a first detection distance (d1) from the illumination zone;
   • a second detection zone ($14_2$), extending to a second detection distance (d2) from the illumination zone, the second detection distance being greater than the first detection distance;
   • a third detection zone ($14_3$), extending to a third detection distance (d3) from the illumination zone, the third detection distance being greater than the second detection distance;

- a processing unit (18), programmed to implement the steps (ii), (iii), (v) and (vii) of a method according to any one of the preceding claims from detection signals formed by the photodetector:

   • in the step (ii), from photons detected in the second and third detection zones;
   • in the step (v), from photons detected in the first detection zone;
   • in the step (vii), from photons detected in the second and third detection zones.

8. Device according to Claim 7, wherein

- the first detection distance is less than 2 cm;
- the second and third detection distances are greater than 2 cm.

**Fig. 1A**

**Fig. 1B**

```
        ┌──────────────┐
        │     100      │
        └──────┬───────┘
  μₐ(L1,t1)    │   μₐ(L2,t1)
               ▼
        ┌──────────────┐
        │     110      │
        └──────┬───────┘
  DPF(d1, t1)  │
               ▼
        ┌──────────────┐
        │     120      │
        └──────┬───────┘
               │   Δμₐ(L1,t1,t2)
               ▼
        ┌──────────────┐
        │     130      │
        └──────┬───────┘
               │  μₐ(L2,t2)
               ▼
```

$\mu_a(L1, t1)$   $\mu_a(L2, t1)$

$DPF(d1, t1)$

$\Delta\mu_a(L1, t1, t2)$

$\mu_a(L2, t2)$

**Fig. 2**

$\mu_a$ cm$^{-1}$

**Fig. 3**

(cm)

**Fig. 4**

Fig. 5A

Fig. 5B

**Fig. 5C**

**Fig. 5D**

Fig. 6A

Fig. 6B

EP 4 394 360 B1

**Fig. 6C**

**Fig. 6D**

**Fig. 7A**

**Fig. 7B**

EP 4 394 360 B1

Fig. 7C

Fig. 7D

27

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2762064 A
- EP 3054282 A
- EP 3054281 A
- EP 3311138 A
- US 9433352 B2